# EUROPEAN PATENT SPECIFICATION

(11) **EP 0 669 977 B1**
(45) Date of publication and mention of the grant of the patent: **03.05.2000**
(21) Application number: 93924544.5
(22) Date of filing: 29.10.1993
(51) Int. Cl.: C12N 15/12, A61K 48/00, C12N 5/10, C12N 15/85, C12N 15/86, A61K 38/17, A01K 67/027

(54) **TRANSFORMED ENDOTHELIAL CELLS**
TRANSFORMIERTE ENDOTHELZELLEN
CELLULES ENDOTHELIALES TRANSFORMEES

(30) Priority: 02.11.1992 GB 9222931
(43) Date of publication of application: 06.09.1995
(73) Proprietor: Novartis AG, 4058 Basel (CH); Novartis-Erfindungen Verwaltungsgesellschaft m.b.H., 1230 Wien (AT)
(72) Inventor: BACH, Fritz, H., Manchester-by-the-Sea, MA 01944 (US); DE MARTIN, Rainer, A-1120 Vienna (AT)
(86) International application number: EP9303015
(87) International publication number: WO9410305

(56) References cited:
- WO-A-89/08147
- WO-A-92/07573
- CELL. vol. 65 , 28 June 1991 , CAMBRIDGE, NA US pages 1281 - 1289 HASKILL ET AL. 'Characterization of an immediate-early gene induced in adherent monocytes that encodes IkappaB-like activity' cited in the application
- EMBO JOURNAL. vol. 12, no. 7 , 1 July 1993 , EYNSHAM, OXFORD GB pages 2773 - 2779 DE MARTIN ET AL. 'Cytokine-inducible expression in endothelial cells of an IkappaBalfa-like gene is regulated by NFkappaB'

## Description

This invention relates to xenotransplantation and in particular to endothelial cells which have been altered to render tissue containing them less likely to be rejected by a recipient in xenotransplantation. More specifically the invention relates to a factor which suppresses the activity of the transcription factor NFκB and its use to inhibit the activation of endothelial cells and suppress the inflammatory process.

A major problem in the successful transplantation of organs between discordant species is hyperacute rejection of the organ. There are two main components involved in the occurrence of hyperacute rejection: the presence of antibodies specific for the xenogeneic cells and the subsequent fixation and activation of the complement system in the recipient. A solution to this problem is proposed in WO 91/05855 which describes transgenic animals that express the complement restriction factors of the recipient. In this way, when an organ from a transgenic animal is grafted into a recipient, activation of the complement pathway in the recipient is blocked by the restriction factor.

However the cells of the donor organ also give rise to rejection by stimulating coagulation in the recipient and by changes in the endothelium of the donor organ. During inflammation, the expression of a number of different genes is up-regulated in endothelial cells, including those coding for interleukins, transcription factors, adhesion molecules, and components of the coagulation system. Transcription of many of these genes involves the transcription factor NFκB.

The transcription factor NFκB is constitutively expressed in the cytoplasm of cells. It has been proposed that the induction of gene transcription by NFκB-like proteins is the result of post-translational modifications involving the translocation of the preformed transcription factor from the cytoplasm to the nucleus. This translation is controlled by the modification of an inhibitor protein called IκB, which binds to and forms a complex with NFκB and thereby holds it in the cytoplasm. Stimulation of the cell by appropriate signals lead to modification of IκB which in turn results in its dissociation from NFκB.

It is believed that binding of the IκB protein to NFκB masks the nuclear localization signal (NLS) of NFκB. Upon stimulation of the cell with specific agents, which depend on the cell type and stage of cell development, IκB is modified in a way that disables binding to NFκB, leading to dissociation of NFκB from IκB. Signals leading to this modification are believed to involve the generation of oxygen radicals and to lead to phosphorylation of IκB at specific sites. As a result the NLS is unmasked and NFκB is translocated to the nucleus, where it binds to NFκB sites. This ultimately leads to transcription of genes involved in the inflammatory process.

The transcription factor NFκB was originally isolated from mature B cells where it binds to a decameric sequence motif in the κ light chain enhancer. Although NFκB was initially believed to be specific for this cell type and this stage of cell development, NFκB-like proteins have since been identified in a large number of cell types and, as discussed above, have been shown to be more generally involved in the induction of gene transcription. This has been further supported by the identification of functionally active NFκB binding sites in several inducible genes. (Baeuerle, P.; 1991; BBA, 1072, p63 to 80).

NFκB is a heterodimeric protein consisting of a 50 kD subunit (p50) and a 65 kD subunit (p65). The cDNAs for p50 and p65 have been cloned and have been shown to be homologous over a region of 300 amino acids. The p50 subunit shows significant homology to the products of the c-rel protooncogene isolated from mammals and birds, and to the Drosophila gene product of dorsal. Recently an additional member of the NFκB family, relB, has been cloned as an immediate early response gene from serum-stimulated fibroblasts.

Both p50 and p65 are capable of forming homodimers, although with different properties: whereas p50 homodimers have strong DNA binding affinity but cannot transactivate transcription, the p65 homodimers can only weakly bind to DNA but are capable of transactivation. p50 is synthesized as the amino-terminal part of a 110 kD precursor (p110), which has no DNA binding and dimerisation activity. The carboxy-terminal part contains eight ankyrin repeats, a motif found in several proteins involved in cell cycle control and differentiation. Cloning of a shorter (2.6 kb) RNA species which is induced in parallel with the 4 kb p50 precursor RNA has revealed that, either by alternative splicing or by differential promoter usage, the C-terminal part of the 110 kD protein can also be expressed independently.

Three IκBα-like proteins have been cloned: pp40, identified as a rel-associated phosphoprotein in transformed chicken lymphoid cells (Davies et al., Science 253 (1991), 1268-1271); RL/IF-1 which is regenerating liver inhibitory factor 1 (Tewarl et al., Mol. Cell. Biol. 12(6) (1992), 2898-2908) and MAD-3 which is the product of a gene induced in human macrophages upon adherence to plastic surfaces (Haskill et al., Cell 65 (1991), 1281-1289).

All three IκBα-like proteins contain five ankyrin repeats. RL/IF-1 has been cloned and shown to be expressed in regenerating liver within 30 minutes after hepatectomy. Deletion mutagenesis studies have revealed that four out of the five ankyrin repeats of pp40 are essential to inhibit DNA binding activity and to associate with c-rel, and that also the C-terminal region is required. Studies with monospecific antibodies, conducted with the 110 kD p50 precursor, have demonstrated that the C-terminal part (the part with IκB activity) masks the nuclear localization signal (NLS) located in the amino-terminal region of p50.

Surprisingly we have now found that IκB expression is induced in endothelial cells (EC) by the same stimuli that activate the EC, although with delayed kinetics as compared to the rapid post-translational activation of NFκB. Expression of IκB therefore appears to represent a naturally occurring feedback mechanism to inhibit EC activation. We have utilized this finding to develop a further strategy to prevent transplant rejection.

Accordingly in a first aspect this invention provides an endothelial cell comprising
i) a heterologous DNA sequence which is constitutively expressed by the cell and codes for expression of a protein having IkB activity, or
ii) a heterologous inducible promoter DNA sequence operably linked with a DNA sequence which codes for expression of a protein having IkB activity , wherein the protein having IkB activity comprises an amino acid sequence which is at least 70% homologous to the amino acid sequence from position number 1 to position number 280 of SEQ ID No. 1, or a part thereof which has IkB activity, such that NFkB mediated induction of gene transcription in the cells is prevented and tissue containing the cells is rendered less likely to be rejected by a transplant recipient than tissue containing endothelial cells which do not comprise the heterologous DNA sequence or the heterologous inducible promoter DNA sequence.

For the purposes of the present description "a protein having IkB activity" is a protein which is able to bind to NFκB and prevent NFκB from migrating to the nucleus and/or binding to NFκB binding sites in the genetic material of a cell and thereby prevents NFκB mediated induction of gene transcription. Such a protein may be a naturally occurring IkB-like protein, part thereof or an analogue or variant of either of these which has this NFκB binding and gene transcription induction inhibiting activity. In a preferred embodiment the protein may be a variant comprising one or more signal sequences adapted to direct the protein to a specific cellular compartment, such as the nucleus of the cell.

Preferably the endothelial cells of the invention express the protein having IkB activity constitutively (i.e. continuously and without delayed kinetics as compared with NFκB expression), immediately following endothelial cell activating stimuli or on demand in response to a predetermined external stimulus.

Typically the endothelial cells of the invention are obtained as the result of the modification of the genetic material of the cells. This modification includes introduction of heterologous DNA coding for the expression of a protein having IkB activity as well as alteration or replacement of the expression control signals of native IkB coding sequences.

Thus in one embodiment the endothelial cell of the invention comprises a heterologous DNA sequence which is constitutively expressed by the cell and codes for expression of a protein having IkB activity.

The IkB activity protein coding sequence is operably linked with a promoter sequence, which is typically also heterologous to the cells, such that the protein is expressed constitutively.

In an alternative embodiment the endothelial cell of the invention comprises a heterologous inducible promoter DNA sequence operably linked with a DNA sequence which codes for expression of a protein having IkB activity, such that the protein is expressed immediately following endothelial cell activating stimuli or on demand in response to a predetermined external stimulus.

The protein coding sequence which is operatively linked with the heterologous inducible promoter sequence, may be a native IkB coding sequence or a heterologous IkB coding sequence and may include sequence coding for an appropriate signal sequence, e.g. a nucleus specific signal sequence. The endothelial cell activating stimuli may be any of the stimuli which give rise to changes in the endothelium of donor tissue or organs and stimulate coagulation in the recipient, leading to rejection. The predetermined stimulus may be the presence of a drug, cytokine or other inducing agent which stimulates expression from the inducible promoter. For example a transplant patient may be treated with an inducing agent, such as a drug, following transplantation to induce IkB expression and thereby block activation of the endothelial cells of the donor organ and consequent rejection of the organ.

Any suitable inducible promoter may be used. For example an inducible promoter such as that described by Gosen and Bujard [PNAS (1992), 89, 5547-5551] may be used.

In a further aspect the invention provides a process for perparing endothelial cells according to the invention comprising transfecting endothelial cells with a heterologous DNA sequence which is constitutively expressed by the cells and codes for expression of a protein having IkB activity.

Moreover in a yet further aspect the invention provides a process for preparing endothelial cells according to the invetion comprising operably linking a heterologous inducible promoter DNA sequence with a native DNA sequence which codes for expression of a protein having IkB activity such that the protein is expressed immediately following endothelial cell activating stimuli or on demand in response to a predetermined external stimulus.

Preferably the heterologous DNA sequences are incorporated in the genome of the cell. Alternatively the heterologous sequences may be maintained in the cell extrachromosomally, either stably or for a limited period.

In accordance with the present invention a previously unknown IkB-like protein, hereinafter referred to as ECI-6, has been identified in porcine aortic endothelial cells (PAEC) stimulated with inflammatory agents such as TNFα and IL-1α. This protein and parts thereof having IkB activity and analogues thereof are included within the scope of the invention. The amino acid sequence of, and natural DNA sequence which codes for, ECI-6 are given in SEQ ID No. 1. The protein having IkB activity may comprise ECI-6 or active parts thereof or active analogues or variants of these.

Thus in a preferred embodiment the heterologous sequence which codes for the protein having IκB activity codes for a protein which comprises a sequence which is at least 70%, preferably at least 80% and more preferably at least 90%, homologous to the protein sequence from position no. 1 to position no. 280 of SEQ ID No. 1 or 2, or part thereof which has IkB activity.

In a particularly preferred embodiment the heterologous sequence codes for a protein sequence which is altered to disable one or more of the phosphorylation sites (amino acids 30 to 36 amino acids 39 to 47 and amino acids 260 to 264 of SEQ ID No. 1). These alterations may include changes of the central Ser in the first and third sequences to Gly and the Tyr in the second sequence to Phe.

The protein with IκB-like activity prevents migration of NFkB to the nucleus and/or inhibits binding of the NFκB to NFκB binding sites in the genetic material of the cell. Inhibition of NFκB binding may be determined using standard bioassay methods. For example an electrophoretic mobility shift assay may be used to detect the inhibition of NFκB binding in endothelial cells. A particular example of such an assay is as follows:

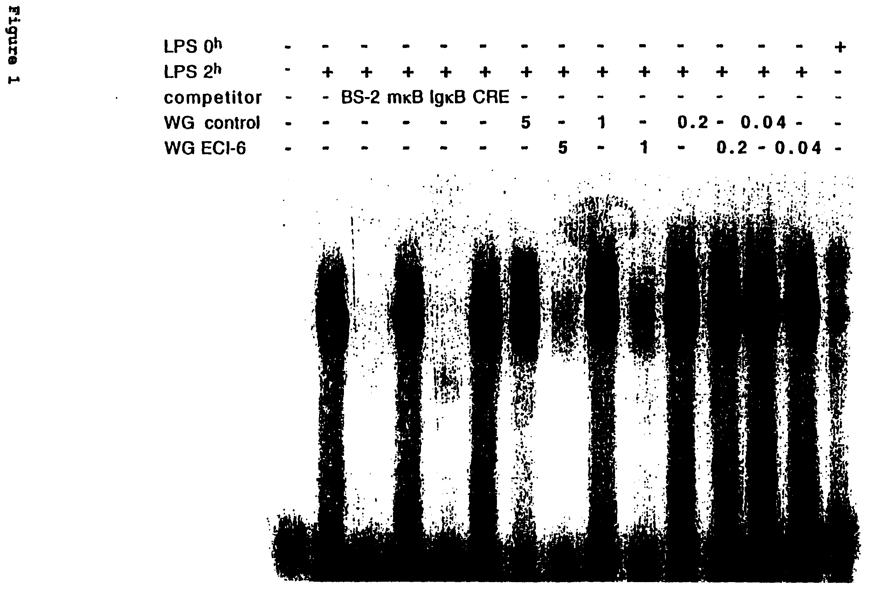

Porcine aortic endothelial cells are grown in a culture medium comprising Dulbecco's Modified Eagle's Medium containing 10 % fetal calf serum. 100 ng of bacterial lipopolysaccharide is used to stimulate the cells for two hours and nuclear proteins are then extracted as described in Dignam et al; 1983; Nucl. Acids Res.; 11, 1475-1489. RNA is then transcribed in vitro from a plasmid (Bluescript II obtained from Stratagene) containing a BamHI-Xhol fragment (bases 126 to 1605) of the cDNA shown in figure 1. The transcription reaction is run according to the manufacturer's protocol except that 0.25 nM m⁷G(5')ppp(5')G (Boehringer) is added.

The RNA obtained is then used for in vitro translation in wheat germ extract or rabbit reticulocyte lysate; both obtained from Promega Corporation, Madison, WI, USA in accordance with the manufacturer's protocol. EMSA is then performed by preparing a labelled 16-mer BS-2 oligonucleotide (given hereinafter as SEQ ID No. 3), which is a NFκB binding site. The binding reaction is conducted at 20°C and at a pH of 7.9 for 15 minutes in a total volume of 15 µl containing 12% glycerol, 12 mM HEPES, 4 mM Tris, 60 mM KCl, 1 mM EDTA, 1 mM DTT, 200 µg/ml poly(dl-dC), 300 µg/ml bovine serum albumin, 0.2 ng of the labelled BS-2 (100.000 cpm), 5 µg nuclear proteins and varying amounts of the translated protein. The inhibition of NFκB binding to the BS-2 oligonucleotide can be determined by comparison to control wheat germ extracts or rabbit reticulocyte lysates.

In further aspects the invention provides donor tissue or a donor organ containing the endothelial cells. The donor material may be xenogeneic for the recipient or may be from the same species. Preferably the donor material is obtained from a pig.

The donor material may be obtained from a transgenic animal. Alternatively the heterologous DNA may be transfected into the endothelial cells of the donor organ by conventional gene therapy techniques. Suitable techniques are described in Miller, A. D.; 1992; Nature, 357, 455-460.

In addition to providing donor organs and tissue the invention may also be generally used to provide genetically altered endothelial cells which express IκB as described above and elsewhere in this description, for instance in relation to disorders of endothelial cells which may benefit from blocking of NFκB mediated induction of expression.

In yet another aspect the invention provides a nucleotide sequence encoding a protein having IκB activity and including the amino acid sequence given in SEQ ID No. 1, starting at amino acid position No 1 and ending at amino acid position No 280; or amino acid sequences which are substantially i.e. at least 95%, homologous thereto; for use in transfected endothelial cells. The transfected endothelial cells may be used in transplantation or to control expression in smooth muscle cells.

The nucleotide sequences may further comprises codons coding for amino acids 281 to 314 of the sequence illustrated in figure 1. Preferably the nucleotide sequences encode a protein in which the sequence is altered to disable one or more of the phosphorylation (amino acids 30 to 36, amino acids 39 to 47 and amino acids 260 to 264).

The alterations preferably result in the changing of the central Ser in the first and third sequence to Gly and the changing of the Tyr in the second sequence to Phe.

In a further aspect the invention provides a DNA sequence which codes for a protein having IkB activity which comprises a heterologous signal sequence.

The signal sequence in typically a signal sequence adapted to direct the protein to a specific cellular compartment, preferably a nucleus specific signal sequence.

In further aspects the invention also provides a recombinant DNA construct comprising a DNA sequence which codes for a protein having IκB activity having operably linked thereto
1) an inducible promoter or
2) an endothelial cell specific promoter,
   wherein the promoter is other than the promoter which is native to said IκB activity protein encoding DNA sequence.

Preferably the promoter is both inducible and endothelial cell specific.

Normally the IκB activity protein encoding sequence is a heterologous sequence and may be a sequence as described above comprising a signal sequence or in relation to SEQ ID No. 1.

The invention further provides an expression vector containing a nucleotide sequence or recombinant DNA construct as defined above.

Preferably the vector is selected from a retroviral vector, an adenoviral vector, a complex of a nucleotide sequence and a transferrin-polycation conjugate and a plasmid formulated in a liposome.

Suitable retroviral vectors are described in WO 92/07573. Suitable adenoviral vectors are described in Rosenfeld, M. A. et al; 1991; Science; **252**, 432. Suitable transferrin-polycation complexes are described in Wagner, E. et al; 1991; Proc. Natl. Acad. Sci. USA, **88**, 4255-4259. Liposome complexes are described in Stewart, M. et al; 1992; Human Gene Therapy, **3**, 267-275.

In another aspect the invention provides a protein having IκB-like activity and comprising the amino acid sequence illustrated in figure 1, starting at amino acid position No 1 and ending at amino acid position No 280.

The protein may further comprise amino acids 281 to 314 of SEQ ID No. 1. These amino acids contain PEST sequences which have been reported in other proteins to be responsible for protein instability (Rogers et al; 1986; Science, **234** 364-368). Preferably these amino acids are not present in the protein and this may be done by introducing a stop codon after the codon for amino acid 280. This may be achieved by mutating the codon for proline at amino acid position 281 by polymerase chain reaction.

Preferably the amino acid sequence of the protein is altered to disable one or more of the phosphorylation sites (amino acids 30 to 36 amino acids 39 to 47 and amino acids 260 to 264).

These alterations may comprise changing the central Ser in the first and third sequence to Gly and changing the Tyr in the second sequence to Phe.

In a further aspect the invention provides a protein having IκB activity which comprises a heterologous signal sequence, e.g. a nucleus specific signal sequence.

It will be appreciated that the endothelial cells and donor organs defined above have a supplementary function in the prevention of transplant rejection in xenotransplantation since the primary rejection is hyperacute rejection. Therefore the genetic material of the cells of the donor organ is typically also altered such that activation of the complement pathway in the recipient is prevented. As described in WO 91/05855 this may be done by providing transgenic animals that express the complement restriction factors of the recipient species. The endothelial cells of a donor organ obtained from such an animal can be modified by gene therapy techniques to provide the endothelial cells defined above. Alternatively a vector as defined above can be introduced into the transgenic animal at the single cell stage or early morula stage. In this way the resulting transgenic animal will express the complement restriction factors and will have endothelial cells as defined above.

Thus in a further aspect the invention also provides non human endothelial cells, tissue, donor organs as defined above which expresses one or more human complement restriction factors.

RNA coding for the protein ECI-6 was isolated from porcine aortic endothelial cells (PAEC) that were stimulated with the inflammatory agents TNFα and IL-1α. It was found that the ECI-6 RNA was induced within 2 hours of stimulation with the inflammatory agents, declining after 4 hours. A small amount was also detected in "unstimulated" cells. Addition of cycloheximide together with LPS for six hours resulted in an augmentation of the signal, suggesting that prior protein synthesis is not required for induction. The RNA was identified by using a differential screening approach using activated versus non-activated endothelial cells.

The IκB activity of ECI-6 was tested in an in vitro DNA binding assay and was found to specifically, and in a dose-dependent manner, inhibit NFκB binding activity present in the nuclear fractions of lipopolysaccharide (LPS)-stimulated PAEC. Similarly, endogenous NFκB present in rabbit reticulocyte lysates was inhibited by priming the lysates with ECI-6 RNA, but not by control lysates. The binding of the NFκB was specific since it could be competed with unlabelled oligonucleotides that represent NFκB binding sites but not with mutated NFκB binding sites or an unrelated oligonucleotide.

The protein ECI-6 shows strong homology (over 90%) to the IκB protein MAD-3 and high homology to the proteins pp40 and RL/IF-1.

Embodiments of the invention are now described, by way of example only, with reference to the drawings in which:
Figure 1 shows a polyacrylamide gel which illustrates the inhibition of binding of nuclear proteins to NFκB sites in the presence of in vitro translated ECI-6 in a wheat germ extract and,
Figure 2 shows a polyacrylamide gel which illustrates the inhibition of the binding of endogenous NFκB to the IκB-2 binding site in rabbit reticulocyte lysates.

### EXAMPLES

### Example 1: Cloning of induced genes

### a) Preparation of a cDNA library of stimulated porcine aortic endothelial cells (PAEC)

Post-confluent PAEC are treated with a combination of human recombinant TNFα and IL-1α (obtained from Genzyme), each at a concentration of 100 U/ml, for 4 and for 9 hours. Cells are harvested at both time points and total cellular RNA for each time point is extracted and isolated using the procedure described in Maniatis et al; 1989; Molecular Cloning, Cold Spring Harbor, Pages 7 to 10. The RNAs from both time points are then pooled. 5 µg of poly A+ RNA is prepared with oligo dT cellulose (Boehringer Mannheim) according to the manufacturer's protocol.

A cDNA library in the bacteriophage λ Zap II is prepared using a cDNA synthesis kit (Stratagene, La Jolla, California) according to the manufacturer's protocol. Packaging of the vectors is then done using the Gigapack Gold system (Stratagene, La Jolla, California). About 2,2 million primary clones are obtained. Approximately 5000 plaques are screened.

### b) Preparation of probes and screening

Two probes are prepared: one from RNA obtained from PAEC that was not induced and one from induced PAEC as described in a). The uninduced probe is prepared by reverse transcribing 1 µg RNA with reverse transcriptase (Gibco-BRL) according to the manufacturer's protocol. 50 µCi of ³²P-CTP is used in the reaction. The induced probe is prepared in the same way, but is then subtracted twice with 10 µg of biotinylated uninduced RNA. The biotinylation and subtraction is done using Subtractor I (InVitrogen, San Diego, California) according to the manufacturer's protocol.

Two replicas from the PAEC library are screened, one with the uninduced probe and the other with the induced probe under standard hybridization conditions at 65°C (Maniatis et al). Phages that give stronger signals with the induced probe as compared to the uninduced probe are subcloned by in-vivo excision using the Stratagene protocol.

In all cases, Northern analysis is carried out according to standard methods (Maniatis et al). A 1.5 kb EcoRI-Xhol fragment of the induced clone that is labelled with a oligo random priming kit (Stratagene) according to the manufacturer's protocol, is used as a probe.

Nucleotide sequencing of the cDNA clones is then determined by dideoxy chain termination method using a Sequenase kit (US Biochemicals, Cleveland, Ohio) according to the manufacturer's protocol. The nucleotide sequence and the predicted amino acid sequence is given in SEQ ID NO: 1. The five ankyrin repeats are at amino acid residues 73 to 99, 110 to 136, 143 to 169, 182 to 208 and 216 to 242, and the three potential CK-II TYR and PKC phosphorylation sites are at amino acid residues 30 to 36, 39 to 47 and 260 to 264 respectively. The 5' part of the sequence (pos. 1-270) is obtained from a genomic clone and is ligated to the cDNA at the Xhol site (pos. 285) to obtain a functional clone for in vitro translations.

The protein sequence is then compared to the sequences of MAD-3, RL/IF-1 and pp40 using the PILEUP program of the UWGCG software and found to have high homology with these proteins. The protein is termed ECI-6.

### Example 2: In-vitro translations

A linearized vector (10 µg; Bluescript) with an insert containing a nucleotide sequence corresponding to positions 126 to 1605 of SEQ ID NO: 1 is transcribed into RNA using T7 polymerase (Stratagene), in a total volume of 100 µl as recommended by the manufacturer, but also including 0.25 mM m⁷G(5')ppp(5')G. The capped mRNA is then extracted in phenol and chloroform and precipitated in ethanol. 1/50 of the product is used for in vitro translation in wheat germ extract or in rabbit reticulocyte lysate, as recommended by the manufacturer (Promega). Different concentrations corresponding to 5, 1, 0.2 and 0.04 µl of the in vitro translation was used in electrophoretic mobility shift analysis (EMSA).

### Example 3: EMSA

The EMSA protocol that is used is that described in Ausubel, I and Frederick, M. (Eds.); 1987; "Current Protocols in Molecular Biology", J. Wiley & Sons, section 12-2. Endothelial cells (EC's) are cultivated in Dulbecco's Modified Eagle's Medium containing 10% fetal calf serum. The endothelial cells are stimulated with 100 ng/ml of bacterial lipopolysaccharide (LPS) for two hours and the nuclear proteins are then extracted as described in Dignam, J.D., Lebovits, R.M. and Roeder, R.G.; 1983; Nucl. Acids Res.; 11, 1475-1489). 0.2 ng of a double stranded 26-mer BS-2 oligonucleotide (the sequence of which is given hereinafter as SEQ ID No. 4; 100.000 cpm) is labelled by filling in the overhangs with the Klenow fragment of DNA-polymerase I in the presence of radioactive nucleoside triphosphates. The oligonucleotide is then incubated in 15 µl binding buffer comprising 12% glycerol, 12 mM HEPES, 4 mM Tris, 60 mM KCl, 1 mM EDTA, 1 mM DTT, 200 µg/ml poly(dl-dC), and 300 µg/ml bovine serum albumin. 15 µg of nuclear proteins and 5, 1, 0.2 or 0.04 µl of the translated ECI-6 protein are then added. The binding reaction is conducted at 30°c and at a pH of 7.9 for 15 minutes. The inhibition of NFκB binding to the BS-2 oligonucleotide can be determined by comparison to control wheat germ extracts or rabbit reticulocyte lysates.

The procedure is repeated with rabbit reticulocyte lysates with or without ECI-6 RNA, but without the nuclear extract.

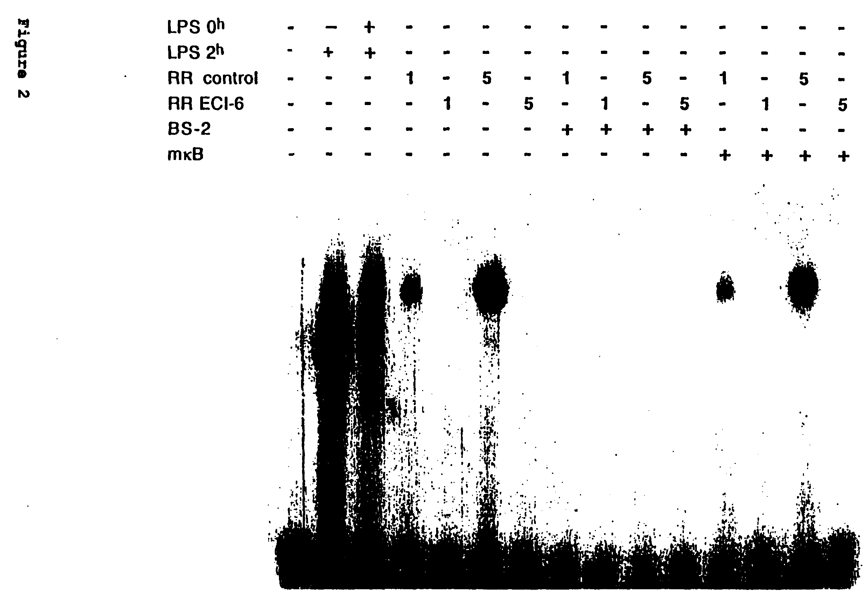

Binding is competed for with either cold BS-2, BS-1 (the sequence of which is given hereinafter as SEQ ID No. 5). ELκB (a binding site from the porcine ELAM-1 promoter having the sequence given hereinafter as SEQ ID No.6) or IgκB (a NFκB binding site from the human immunoglobulin in kappa light chain enhancer having the sequence given hereinafter as SEQ ID No, 7), but not with a mutated NFκB site having the sequence given hereinafter as SEQ ID No. 8. A 500-fold molar excess of unlabelled oligonucleotides is used for competition assays. The resulting complexes are separated on a 5% polyacrylamide gel as described in Dignam et al. The results are illustrated in figures 3a and 3b. It is seen that the protein ECI-6 inhibits the binding of NFκB to the IκB-2 binding site.

### Example 4: Transient Expression Experiments

A minimal NFκB-responsive promoter is constructed in the luciferase reporter gene vector UBT.Luc (de Martin et al., 1993: Gene 124, 137-138) using standard molecular biology techniques. Briefly, a double-stranded oligonucleotide representing 37 bp of the thymidine kinase promoter with EcoRI and HindIII overhangs at the 5' and 3' ends, respectively, is cloned into pKSM13 (Stragene), resulting in the vector pKSM13-TK. Two copies of an double-stranded oligonuceleotide with EcoRI overhangs, representing a NFkB binding site, are cloned into pKSM13-TK, resulting in the vector pKSM13-2xIgkB-TK. Correctness of the constuct is confirmed by DNA sequencing. The 2xIgkB-TK fragment is excised by digestion with NotI and HindIII (A NotI site is located upstream (5') of the EcoRI site). The resultant fragment is ligated into UBT.Luc which has been cut with the corresponding restriction enzymes, resulting in the vector 2xIgkBTK.Luc. The sequence of the The 2xIgkB-TK fragment is given below and in SEQ ID No. 9:

EcoRI          NFkB          EcoRI          NFkB

GAATTCAGAG[?]GGGATTTCCCGAGGAATTCAGAGGGGGATTTCCCGAG-

EcoRI

-GAATTCCACTTCGCATATTAAGGTGACGCGTGTGGCCTCGAA-

HindIII
-CACCGAGCGAAGCTT

This vector is transfected into NIH3T3 cell using Lipofectin (BRL, see attached protocol). Luciferase assays are performed as described (de Wet et al., 1987: Mol. Cell. Biol. 7, 725-737) No expression of luciferase above background is detected. When 2xIgkBTK.Luc is con-transfected together with an expression vector for the p65 subunit of NFkB (CMV.p65), high levels of luciferase are detected. When, in addition to 2xIgkBTK.Luc and CMV.p65, an expression vector for ECI-6/IkBa is co-transfected, levels of luciferase are reduced to background. All transfections contain an RSV.β-galactosidase expression vector as internal control.

The PEST region deletion mutant is generated by PCR by introducing a stop codon after amino acid Leucine 280.

### Example 5: Construction of recombinant retroviruses for stable transduction of primary PAEC with ECI-6/IκB cDNA

A panel of 5 retroviral vectors are evaluated for the production of recombinant retroviruses capable of high efficiency transduction of PAECs (pig aortic endothelial cells) with the human thrombomodulin molecule. The pNTK-2 vector is found to generate the highest titre recombinant viruses and expression in pooled infected PAECs as high as with vectors in which the gene is driven by the 5' viral LTR. On the basis of this study, pNTK-2 is selected for construction of a recombinant retrovirus for expression of the ECI-6/IκB cDNA in PAEC. The recombinant provirus is constructed by insertion of a ca 1kb partial Xhol-Sall fragment comprising the ECI-6 cDNA into the unique Xhol site downstream of the tk promoter in the pNTK-2 provirus. Pure recombinant containing the cDNA in both sense and antisense orientation with respect to the tk promoter, distinguished by BamHI digestion, are recovered in E. coli XL-1 blue. The antisense construct is taken for control purposes. To maximise the efficiency of transfection of the PA317 packaging cell line highly pure plasmid is isolated from bacterial cultures with Qiagen. To minimise the the occurence of integrations incompatible with virus production, sense and antisense constructs (10µg) are linearized with HindIII restriction endonuclease, which cleaves outside of the provirus. Digested DNA is purified by successive phenol-chloroform and chloroform extractions and ethanol precipitation and dissolved in 100µl of electroporation buffer (PBSA + 20mM HEPES buffer, pH 7.0) overnight at 4°C. Resolubilisation is confirmed by agarose gel electrophoresis before transfection. PA317 packaging cells are transfected or mock-transfected (-DNA) using a Biorad electroporator set at 0.25kV and 500 microfarads which generates a time constant of 13.7 millisecs. Surviving cells are resuspended in 20ml of fresh culture medium and incubated overnight before application of selection (0.8mg/ml G418). After 7 days of selection drug-resistant colonies are obtained from both sense and antisense transfected cells whilst control (-DNA) cells are fully killed.

Colonies are pooled and seeded into T-75 flasks in G418-containing medium. Subconfluent log phase cells from on flask are frozen in aliquots in LN₂ (master stock cells). Cells for virus harvesting are grown to confluence and virus harvested into 20mls of complete medium over a 24 hour period. Virus containing medium is then snap-frozen in LN₂ to kill producer cells in suspension and for storage at -80°C. Two additional harvests of virus are then made. Cells are tested for IκB expression and also assayed for inhibition of NFkB associated induction of expression.

SEQUENCE LISTING

(1) GENERAL INFORMATION:

(i) APPLICANT:
(A) NAME: Sandoz Ltd
(B) STREET: P. Box
(C) CITY: Basel
(D) STATE: B S
(E) COUNTRY: Switzerland
(F) POSTAL CODE (ZIP): CH 4002
(G) TELEPHONE: 061 324 1111
(H) TELEFAX: 061 324 7572
(I) TELEX: 965 050 55

(A) NAME: Sandoz - Patent - GmbH
(B) STREET: Humboldstrasse 3
(C) CITY: Lorrach
(D) STATE: Germany
(E) COUNTRY: Germany
(F) POSTAL CODE (ZIP): 2340
(G) TELEPHONE: 0 7621/4 8014

(A) NAME: Sandoz Erfindungen
Verwaltungsgesellschaft
m.b.H.
(B) STREET: Brunner Strasse 59
(C) CITY: Vienna
(D) STATE: Austria
(E) COUNTRY: Austria
(F) POSTAL CODE (ZIP): A-1235 Vienna

(ii) TITLE OF INVENTION: Transformed Endothelial Cells

(iii) NUMBER OF SEQUENCES: 9

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version
#1.25
(EPO)

(v) CURRENT APPLICATION DATA:
APPLICATION NUMBER: WO PCT/EP93/
(vi) PRIOR APPLICATION DATA:
(A) APPLICATION NUMBER: GB 9222931.9
(B) FILING DATE: 02-NOV-1992

(2) INFORMATION FOR SEQ ID NO: 1:

(i) SEQUENCE CHARACTERISTICS:

(A) LENGTH: 1604 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(v) FRAGMENT TYPE: internal

(vi) ORIGINAL SOURCE:
(A) ORGANISM: Sus scrofa

(vii) IMMEDIATE SOURCE:
(B) CLONE: ECI-6

(ix) FEATURE:
(A) NAME/KEY: CDS
(B) LOCATION: 126..1070

(xi) SEQUENCE DESCRIPTION: SEQ ID NO: 1:

GCGCTGCCGA GCCCACAACA GTCCGAGGCC ATCGTCCCGC CCGCCCGAGC CACCGCGAGC 60

AGCCACGCGC CGCGCAGCCT GTGGCCCGCG CACCTAGGGA GCAGCGCCCA AGCCCTCATC 120

GCGCC ATG TTC CAG CCC GCA GAG CCC GGC CAG GAG TGG GCC ATG GAG 167
Met Phe Gln Pro Ala Glu Pro Gly Gln Glu Trp Ala Met Glu
1               5                   10

GGG CCC CGG GAC GCG CTC AAG AAG GAG CGG CTA CTG GAT GAC CGC CAC 215
Gly Pro Arg Asp Ala Leu Lys Lys Glu Arg Leu Leu Asp Asp Arg His
15                  20                  25                  30

GAC AGC GGC CTG GAC TCC ATG AAG GAC GAG GAG TAC GAG CAG ATG GTG 263
Asp Ser Gly Leu Asp Ser Met Lys Asp Glu Glu Tyr Glu Gln Met Val
                35                  40                  45

AAG GAG CTG CGC GAG ATC CGC CTC GAG CCG CAG GAG GCG CCC CGC GGC 311
Lys Glu Leu Arg Glu Ile Arg Leu Glu Pro Gln Glu Ala Pro Arg Gly
            50                  55                  60

GCC GAG CCC TGG AAG CAG CAG CTC ACC GAG GAC GGA GAC TCG TTC CTG 359
Ala Glu Pro Trp Lys Gln Gln Leu Thr Glu Asp Gly Asp Ser Phe Leu
        65                  70                  75

CAC TTG GCC ATC ATC CAT GAA GAG AAG GCA CTG ACC ATG GAA GTG GTC 407
His Leu Ala Ile Ile His Glu Glu Lys Ala Leu Thr Met Glu Val Val
    80                  85                  90

CGC CAA GTG AAG GGA GAT CTG GCT TTT CTT AAC TTC CAG AAC AAC CTG 455
Arg Gln Val Lys Gly Asp Leu Ala Phe Leu Asn Phe Gln Asn Asn Leu
95                  100                 105                 110

CAG CAG ACT CCA CTC CAC TTG GCG GTG ATC ACC AAC CAG CCA GAA ATC 503
Gln Gln Thr Pro Leu His Leu Ala Val Ile Thr Asn Gln Pro Glu Ile
                115                 120                 125

GCT GAG GCA CTT CTG GAA GCT GGC TGT GAT CCT GAG CTC CGA GAC TTT 551
Ala Glu Ala Leu Leu Glu Ala Gly Cys Asp Pro Glu Leu Arg Asp Phe
130 135 136 140

CGA GGA AAT ACC CCT CTA CAC CTT GCC TGT GAG CAG GGC TGC CTG GCC 599
Arg Gly Asn Thr Pro Leu His Leu Ala Cys Glu Gln Gly Cys Leu Ala
143 145 150 155

AGT GTG GGA GTC CTG ACT CAG CCC CGC GGG ACC CAG CAC CTC CAC TCC 647
Ser Val Gly Val Leu Thr Gln Pro Arg Gly Thr Gln His Leu His Ser
    160                 165                 170

ATT CTG CAG GCC ACC AAC TAC AAT GGC CAC ACA TGT CTG CAC TTA GCC 695
Ile Leu Gln Ala Thr Asn Tyr Asn Gly His Thr Cys Leu His Leu Ala
175                 180                 185                 190

TCG ATC CAT GGC TAC CTG GGC ATT GTG GAG CTG TTG GTG TCT TTG GGT 743
Ser Ile His Gly Tyr Leu Gly Ile Val Glu Leu Leu Val Ser Leu Gly
                195                 200                 205

GCT GAT GTC AAC GCT CAG GAG CCC TGC AAT GGC CGA ACC GCC CTG CAT 791
Ala Asp Val Asn Ala Gln Glu Pro Cys Asn Gly Arg Thr Ala Leu His
            210                 215                 220

CTT GCG GTG GAC CTG CAG AAT CCC GAC CTG GTG TCG CTC TTG TTG AAG 839
Leu Ala Val Asp Leu Gln Asn Pro Asp Leu Val Ser Leu Leu Leu Lys
        225                 230                 235

TGT GGG GCT GAT GTC AAC AGA GTC ACC TAC CAG GGC TAC TCC CCG TAC 887
Cys Gly Ala Asp Val Asn Arg Val Thr Tyr Gln Gly Tyr Ser Pro Tyr
    240                 245                 250

CAG CTC ACC TGG GGC CGC CCA AGC ACT CGG ATA CAG CAG CAG CTG GGC 935
Gln Leu Thr Trp Gly Arg Pro Ser Thr Arg Ile Gln Gln Gln Leu Gly
255                 260                 265                 270

CAG CTG ACC CTA GAA AAC CTC CAG ATG CTT CCA GAG AGC GAG GAT GAG 983
Gln Leu Thr Leu Glu Asn Leu Gln Met Leu Pro Glu Ser Glu Asp Glu
                275                 280                 285

GAG AGC TAT GAC ACG GAG TCA GAG TTC ACA GAG GAT GAG CTG CCC TAT 1031
Glu Ser Tyr Asp Thr Glu Ser Glu Phe Thr Glu Asp Glu Leu Pro Tyr
            290                 295                 300

GAC GAC TGC GTG CTT GGA GGC CAG CGC CTG ACG TTA TGA GCTTTGG 1077
Asp Asp Cys Val Leu Gly Gly Gln Arg Leu Thr Leu
305 310 315

AAAGTGTCTA AAAGACCATG TACTTGTACA TTTGTACAAA ATCAAGAGTT TTATTTTTCT 1137

AAAAAAAAAG AAAAAAAGAA AAAAAAAGAA AAAAGGGTAT ACTTATAACC ACACCGCACA 1197

CTGCCTGGCC TGAAACATTT TGCTCTGGTG GATTAGCCCC GATTTTGTTA TTCTTGTGAA 1257

CTTTGGAAAG GCGCCAAGGA GGATCATCGG AATGCAGAGA GAACCTCTTT TAAACGGCAC 1317

CTTGGTGGGG CCTGGGGGAA AGGTTATCCC TAATTTGATG GGACTCTTTT ATTTATTGCG 1377

CTTCTTGGTT GAACCACCAT GGAGTCAGTG GTGGAGCCCA GGTGTATCTG GGAAATGTTA 1437

GAATCAGGTG TGTTGTTAAA CCTGTCAGTG GGGTGGGGTT AAAAGTCACG ACCTGTCAAG 1497

GTTTGTGTTA CCCTGCTGTA AATACTGTAC ATAATGTATT TTGTTGGTAA TTATTTTGGT 1557

ACTTCTAAGA TGTATATTTA TTAAATGGAT TTTTACAAAC AGAAAAA 1604

(2) INFORMATION FOR SEQ ID NO: 2:

(i) SEQUENCE CHARACTERISTICS:
(A) LENGTH: 314 amino acids
(B) TYPE: amino acid
(D) TOPOLOGY: linear

(ii) MOLECULE TYPE: protein

(xi) SEQUENCE DESCRIPTION: SEQ ID NO: 2:

Met Phe Gln Pro Ala Glu Pro Gly Gln Glu Trp Ala Met Glu Gly Pro
1               5                   10                  15

Arg Asp Ala Leu Lys Lys Glu Arg Leu Leu Asp Asp Arg His Asp Ser
            20                  25                  30

Gly Leu Asp Ser Met Lys Asp Glu Glu Tyr Glu Gln Met Val Lys Glu
        35                  40                  45

Leu Arg Glu Ile Arg Leu Glu Pro Gln Glu Ala Pro Arg Gly Ala Glu
    50                  55                  60

Pro Trp Lys Gln Gln Leu Thr Glu Asp Gly Asp Ser Phe Leu His Leu
65                  70                  75                  80

Ala Ile Ile His Glu Glu Lys Ala Leu Thr Met Glu Val Val Arg Gln
                85                  90                  95

Val Lys Gly Asp Leu Ala Phe Leu Asn Phe Gln Asn Asn Leu Gln Gln
            100                 105                 110

Thr Pro Leu His Leu Ala Val Ile Thr Asn Gln Pro Glu Ile Ala Glu
        115                 120                 125

Ala Leu Leu Glu Ala Gly Cys Asp Pro Glu Leu Arg Asp Phe Arg Gly
    130                 135                 140

Asn Thr Pro Leu His Leu Ala Cys Glu Gln Gly Cys Leu Ala Ser Val
145                 150                 155                 160

Gly Val Leu Thr Gln Pro Arg Gly Thr Gln His Leu His Ser Ile Leu
                165                 170                 175

Gln Ala Thr Asn Tyr Asn Gly His Thr Cys Leu His Leu Ala Ser Ile
            180                 185                 190

His Gly Tyr Leu Gly Ile Val Glu Leu Leu Val Ser Leu Gly Ala Asp
        195                 200                 205

Val Asn Ala Gln Glu Pro Cys Asn Gly Arg Thr Ala Leu His Leu Ala
    210                 215                 220

Val Asp Leu Gln Asn Pro Asp Leu Val Ser Leu Leu Leu Lys Cys Gly
225                 230                 235                 240

Ala Asp Val Asn Arg Val Thr Tyr Gln Gly Tyr Ser Pro Tyr Gln Leu
                245                 250                 255

Thr Trp Gly Arg Pro Ser Thr Arg Ile Gln Gln Gln Leu Gly Gln Leu
            260                 265                 270

Thr Leu Glu Asn Leu Gln Met Leu Pro Glu Ser Glu Asp Glu Glu Ser
        275                 280                 285

Tyr Asp Thr Glu Ser Glu Phe Thr Glu Asp Glu Leu Pro Tyr Asp Asp
    290                 295                 300

Cys Val Leu Gly Gly Gln Arg Leu Thr Leu
305                 310

(2) INFORMATION FOR SEQ ID NO: 3:

     (i) SEQUENCE CHARACTERISTICS:
          (A) LENGTH: 16 base pairs
          (B) TYPE: nucleic acid
          (C) STRANDEDNESS: single
          (D) TOPOLOGY: linear

    (ii) MOLECULE TYPE: cDNA

   (iii) HYPOTHETICAL: NO

   (iii) ANTI-SENSE: NO

    (xi) SEQUENCE DESCRIPTION: SEQ ID NO: 3:

AATTCGGCTT GGAAAT                                                  16

(2) INFORMATION FOR SEQ ID NO: 4:

     (i) SEQUENCE CHARACTERISTICS:
          (A) LENGTH: 26 base pairs
          (B) TYPE: nucleic acid
          (C) STRANDEDNESS: single
          (D) TOPOLOGY: linear

    (ii) MOLECULE TYPE: cDNA

   (iii) HYPOTHETICAL: NO

   (iii) ANTI-SENSE: NO

    (xi) SEQUENCE DESCRIPTION: SEQ ID NO: 4:

AATTCGGCTT GGAAATTCCC CGAGCG                                       26

(2) INFORMATION FOR SEQ ID NO: 5:

     (i) SEQUENCE CHARACTERISTICS:
          (A) LENGTH: 27 base pairs
          (B) TYPE: nucleic acid
          (C) STRANDEDNESS: single
          (D) TOPOLOGY: linear

    (ii) MOLECULE TYPE: cDNA

(iii) HYPOTHETICAL: NO

(iii) ANTI-SENSE: NO

(xi) SEQUENCE DESCRIPTION: SEQ ID NO: 5:

AATTCGTCGG GAGGACTTTC CAGCCAG 27

(2) INFORMATION FOR SEQ ID NO: 6:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(xi) SEQUENCE DESCRIPTION: SEQ ID NO: 6:

AATTCATGCT GCTGGGAATT CCTCTGTATG 30

(2) INFORMATION FOR SEQ ID NO: 7:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(xi) SEQUENCE DESCRIPTION: SEQ ID NO: 7:

ATTCAGAGGG GGATTTCCCA GAGG 24

(2) INFORMATION FOR SEQ ID NO: 8:

(i) SEQUENCE CHARACTERISTICS:
(A) LENGTH: 26 base pairs

(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(xi) SEQUENCE DESCRIPTION: SEQ ID NO: 8:

AGCTTAGATT TTACTTTCCG AGAGGA 26

(2) INFORMATION FOR SEQ ID NO: 9:

(i) SEQUENCE CHARACTERISTICS:
(A) LENGTH: 105 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iii) ANTI-SENSE: NO

(xi) SEQUENCE DESCRIPTION: SEQ ID NO: 9:

GAATTCAGAG GGGGATTTCC CGAGGAATTC AGAGGGGGAT TTCCCGAGGA ATTCCACTTC 60

GCATATTAAG GTGACGCGTG TGGCCTCGAA CACCGAGCGA AGCTT 105

## Claims

1. An endothelial cell comprising
i) a heterologous DNA sequence which is constitutively expressed by the cell and codes for expression of a protein having IkB activity, or
ii) a heterologous inducible promoter DNA sequence operably linked with a DNA sequence which codes for expression of a protein having IkB activity, wherein the protein having IkB activity comprises an amino acid sequence which is at least 70 % homologous to the amino acid sequence from position number 1 to position number 280 of SEQ ID No. 1, or a part thereof which has IkB activity, such that NFkB mediated induction of gene transcription in the cells is prevented and tissue containing the cells is rendered less likely to be rejected by a transplant recipient than tissue containing endothelial cells which do not comprise the heterologous DNA sequence of the heterologous inducible promoter DNA sequence.

2. An endothelial cell according to claim 1, in which the DNA sequence which codes for expression of a protein having IkB activity has been altered to disable one or more of the phosphorylation sites at amino acids 30 to 36, 39 to 47, or 260 to 264 of SEQ ID No. 1 by changing Ser at amino acid 32 or amino acid 262 to Gly, or Tyr at amino acid 42 to Phe.

3. Tissue or a donor organ comprising endothelial cells according to claim 1 or 2.

4. Tissue or a donor organ according to claim 3 which is xenogeneic for the recipient.

5. A DNA sequence which codes for expression of a protein having IkB activity and including the amino acid sequence given in SEQ ID No. 1 starting at amino acid position number 1 and ending at amino acid position number 280.

6. A DNA sequence which codes for expression of a protein having IkB activity comprising a sequence which is at least 95% homologous to the sequence given in SEQ ID No. 1 starting at amino acid position number 1 and ending at amino acid position number 280.

7. An expression vector which is a retroviral vector, an adenoviral vector, an endothelial cell specific viral vector, a complex of the recombinant DNA construct to be expressed and a transferrin-polycation conjugate or a plasmid formulated in a liposome and which comprises an endothelial cell specific promoter operably linked with a DNA sequence which codes for expression of a protein having IkB activity, wherein the promoter is other than the promoter which is native to said DNA sequence and the protein having IkB activity comprises an amino acid sequence which is at least 70 % homologous to the amino acid sequence from position No. 1 to position No. 2800 of SEQ ID No. 1, or a part thereof which has IkB activity.

8. A process for the preparation of endothelial cells according to claim 1, comprising transfecting endothelial cells with a heterologous DNA sequence which is constitutively expressed by the cell and codes for expression of a protein having IkB activity which comprises an amino acid sequence which is at least 70 % homologous to the amino acid sequence from position No. 1 to position No. 280 of SEQ ID No. 1, or a part thereof which has IkB activity.

9. A process for the preparation of endothelial cells according to claim 1, comprising operably linking a heterologous inducible promoter DNA sequence with a native DNA sequence which codes for expression of a protein having IkB activity which comprises an amino acid sequence which is at least 70 % homologous to the amino acid sequence from position No. 1 to position No. 280 of SEQ ID No. 1, or a part thereof which has IkB activity.

10. A protein having IkB activity which comprises the amino acid sequence given in SEQ ID No. 1 starting at amino acid position number 1 and ending at amino acid position number 280.

11. A protein according to claim 10, in which the amino acid sequence has been altered to disable one or more of the phosphorylation sites at amino acids 30 to 36, 39 to 47 or 260 to 264 of SEQ ID No. 1 by changing Ser at amino acid 32 or amino acid 262 to Gly, or Tyr at amino acid 42 to Phe.

12. A protein having IkB activity which comprises the amino acid sequence from position No. 1 to position No. 280 of SEQ ID No. 1, or a part thereof which has IkB activity, and which comprises a heterologous nucleus specific signal sequence.

13. Non human endothelial cells according to claim 1 or 2 or tissue or a donor organs according to claim 3 or 4 which express one or more human complement restriction factors, selected from Factor I, Factor H, C4 binding protein, DAF, Membrane Cofactor protein, CR1, CR2, C8bp, P-18 or SP4040.

## Patentansprüche

1. Endothelzelle, die enthält
i) eine heterologe DNA Sequenz, die konstitutiv von der Zelle exprimiert wird und für die Expression eines Proteins mit IκB Aktivität kodiert, oder
ii) eine heterologe induzierbare Promotor DNA Sequenz, die funktionsfähig mit einer DNA Sequenz verbunden ist, die für die Expression eines Proteins mit IκB Aktivität kodiert, worin das Protein mit IκB Aktivität eine Aminosäuresequenz umfaßt, die zumindest zu 70 % homolog zur Aminosäuresequenz von Positionsnummer 1 bis Positionsnummer 280 der SEQ ID Nr. 1 oder einem Teil hiervon mit IκB Aktivität ist, so daß die NFκB vermittelte Induktion der Gentranskription in den Zellen verhindert wird und Gewebe, das die Zellen enthält, unempfindlicher gegenüber einer Abstoßung durch den Transplantatempfänger gemacht wird, als das Gewebe, das Endothelzellen enthält, die die heterologe DNA Sequenz der heterolog induzierbaren Promotor DNA Sequenz nicht enthalten.

2. Endothelzelle nach Anspruch 1, worin die DNA Sequenz, die für die Expression eines Proteins mit IκB Aktivität kodiert, verändert wurde, um eine oder mehrere der Phosphorylierungsstellen an den Aminosäuren 30 bis 36, 39 bis 47 oder 260 bis 264 der SEQ ID Nr. 1 durch den Austausch von Ser an Aminosäureposition 32 oder Aminosäureposition 262 gegen Gly oder Tyr an der Aminosäureposition 42 gegen Phe unbrauchbar zu machen.

3. Gewebe oder Donororgan, das Endothelzellen nach Anspruch 1 oder 2 enthält.

4. Gewebe oder Donororgan nach Anspruch 3, das für den Empfänger xenogen ist.

5. DNA Sequenz, die für die Expression eines Protein mit IκB Aktivität kodiert und die in SEQ ID Nr. 1 angegebene Aminosäuresequenz umfaßt, die an der Aminosäureposition 1 beginnt und und an der Aminosäureposition 280 endet.

6. DNA Sequenz, die für die Expression eines Proteins mit IκB Aktivität kodiert und eine Sequenz umfaßt, die zumindest zu 95 % homolog ist zur in SEQ ID Nr. 1 angegebenen Sequenz, die an der Aminosäureposition 1 beginnt und an der Aminosäureposition 280 endet.

7. Expressionsvektor, der ein Retrovirusvektor, ein Adenovirusvektor, ein Endothelzellen-spezifischer Virusvektor, ein Komplex aus dem zu exprimierenden rekombinanten DNA Konstrukt und einem Transferin-Polykation-Konjugat oder ein Plasmid ist, das in einem Liposom formuliert ist und der umfaßt einen Endothelzellen-spezifischen Promotor funktionsfähig verbunden mit einer DNA Sequenz, die für die Expression eines Proteins mit IκB Aktivität kodiert, worin der Promotor anders als der Promotor ist, der für die DNA Sequenz nativ ist und das Protein mit IκB Aktivität eine Aminosäuresequenz umfaßt, die zumindest zu 70 % homolog zur Aminosäuresequenz von Positionsnummer 1 bis Positionsnummer 280 von SEQ ID Nr. 1 oder einem Teil hiervon ist, der IκB Aktivität aufweist.

8. Verfahren zur Herstellung von Endothelzellen nach Anspruch 1, gekennzeichnet durch Transfektion der Endothelzellen mit einer heterologen DNA Sequenz, die konstitutiv in den Zellen exprimiert wird und für ein Protein mit IκB Aktivität kodiert, das eine Aminosäuresequenz umfaßt, die zumindest zu 70 % homolog zur Aminosäuresequenz von Positionsnummer 1 bis Positionsnummer 280 von SEQ ID Nr. 1 oder einem Teil hiervon ist, der IκB Aktivität aufweist.

9. Verfahren zur Herstellung von Endothelzellen nach Anspruch 1, gekennzeichnet durch funktionsfähiges Verbinden einer heterolog induzierbaren Promotor DNA Sequenz mit einer nativen DNA Sequenz, die für die Expression eines Proteins mit IκB Aktivität kodiert und eine Aminosäuresequenz umfaßt, die zumindest zu 70 % homolog zur Aminosäuresequenz von Positionsnummer 1 bis Positionsnummer 280 von SEQ ID Nr. 1 oder einem Teil hiervon ist, der IκB Aktivität aufweist.

10. Protein mit IκB Aktivität, das die in SEQ ID Nr. 1 angegebene Aminosäuresequenz umfaßt, die bei Aminosäurepositionsnummer 1 beginnt und bei Aminosäurepositionsnummer 280 endet.

11. Protein nach Anspruch 10, worin die Aminosäuresequenz verändert wurde, um eine oder mehrere der Phosphorylierungsstellen an den Aminosäuren 30 bis 36, 39 bis 47 oder 260 bis 264 der SEQ ID Nr. 1 durch den Austausch von Ser an Aminosäureposition 32 oder Aminosäureposition 262 gegen Gly oder Tyr an der Aminosäureposition 42 gegen Phe unbrauchbar zu machen.

12. Protein mit IκB Aktivität, das die Aminosäuresequenz von Positionsnummer 1 bis Positionsnummer 280 der SEQ ID Nr. 1 oder einen Teil hiervon mit IκB Aktivität und eine heterologe kernspezifische Signalsequenz umfaßt.

13. Nicht-humane Endothelzelle nach Anspruch 1 oder 2 oder Gewebe oder Donororgane nach Anspruch 3 oder 4, die einen oder mehrere humane Komplementrestriktionsfaktoren exprimieren, ausgewählt aus Faktor I, Faktor H, C4 Bindungsprotein, DAF, Membrancofaktorprotein, CR1, CR2, C8bp, P-18 oder SP4040.

## Revendications

1. Cellule endothéliale comprenant
i) une séquence d'ADN hétérologue qui est constitutivement exprimée par la cellule et code pour l'expression d'une protéine ayant une activité d'IkB, ou
ii) une séquence d'ADN promoteur inductible hétérologue liée de manière opérationnelle à une séquence d'ADN qui code pour l'expression d'une protéine ayant une activité d'IkB, où la protéine ayant une activité d'IkB comprend une séquence d'acides aminés qui est homologue à au moins 70 % à la séquence d'acides aminés allant de la position N^{o} 1 à la position N^{o} 280 de SEQ ID N^{o} 1, ou une de ses parties qui a une activité d'IkB, de manière que l'induction, se faisant par la médiation de NFkB, de la transcription du gène dans les cellules soit empêchée et que le tissu contenant les cellules soit rendu moins susceptible d'être rejeté par un receveur de greffe qu'un tissu contenant des cellules endothéliales qui ne comprennent pas la séquence d'ADN hétérologue ou la séquence d'ADN promoteur inductible hétérologue.

2. Cellule endothéliale selon la revendication 1, dans laquelle la séquence d'ADN qui code pour l'expression d'une protéine ayant une activité d'IkB a été modifiée pour désactiver un ou plusieurs des sites de phosphorylation au niveau des acides aminés 30 à 36, 39 à 47, ou 260 à 264 de SEQ ID N^{o} 1 en changeant la Ser au niveau de l'acide aminé 32 ou de l'acide aminé 262 en Gly, ou la Tyr au niveau de l'acide aminé 42 en Phe.

3. Tissu ou organe donneur comprenant des cellules endothéliales selon la revendication 1 ou 2.

4. Tissu ou organe donneur selon la revendication 3 qui est xénogénique pour le receveur.

5. Séquence d'ADN qui code pour l'expression d'une protéine ayant une activité d'IkB et qui inclut la séquence d'acides aminés donnée dans SEQ ID N^{o} 1 commençant à l'acide aminé en position N^{o} 1 et se terminant à l'acide aminé en position N^{o} 280.

6. Séquence d'ADN qui code pour l'expression d'une protéine ayant une activité d'IkB et comprenant une séquence qui est homologue à au moins 95 % à la séquence donnée dans SEQ ID N^{o} 1 commençant à l'acide aminé en position N^{o} 1 et se terminant à l'acide aminé en position N^{o} 280.

7. Vecteur d'expression qui est un vecteur rétroviral, un vecteur adénoviral, un vecteur viral spécifique des cellules endothéliales, un complexe du produit d'assemblage recombinant à exprimer et d'un conjugué transferrine-polycation ou un plasmide formulé dans un liposome et qui comprend un promoteur spécifique des cellules endothéliales lié de manière opérationnelle à une séquence d'ADN qui code pour l'expression d'une protéine ayant une activité d'IkB, où le promoteur est différent du promoteur qui est natif pour ladite séquence d'ADN et la protéine ayant une activité d'IkB comprend une séquence d'acides aminés qui est homologue à au moins 70 % à la séquence d'acides aminés allant de la position N^{o} 1 à la position N^{o} 280 de SEQ ID N^{o} 1, ou une de ses parties qui a une activité d'IkB.

8. Procédé de préparation de cellules endothéliales selon la revendication 1, dans lequel on transfecte des cellules endothéliales avec une séquence d'ADN hétérologue qui est constitutivement exprimée par la cellule et code pour l'expression d'une protéine ayant une activité d'IkB qui comprend une séquence d'acides aminés qui est homologue à au moins 70 % à la séquence d'acides aminés allant de la position N^{o} 1 à la position N^{o} 280 de SEQ ID N^{o} 1, ou une de ses parties qui a une activité d'IkB.

9. Procédé de préparation de cellules endothéliales selon la revendication 1, dans lequel on lie de manière opérationnelle une séquence d'ADN promoteur inductible hétérologue avec une séquence d'ADN native qui code pour l'expression d'une protéine ayant une activité d'IkB qui comprend une séquence d'acides aminés qui est homologue à au moins 70 % à la séquence d'acides aminés allant de la position N^{o} 1 à la position N^{o} 280 de SEQ ID N^{o} 1, ou une de ses parties qui a une activité d'IkB.

10. Protéine ayant une activité d'IkB qui comprend la séquence d'acides aminés donnée dans SEQ ID N^{o} 1 commençant à l'acide aminé en position N^{o} 1 et se terminant à l'acide aminé en position N^{o} 280.

11. Protéine selon la revendication 10, dans laquelle la séquence d'acides aminés a été modifiée pour désactiver un ou plusieurs des sites de phosphorylation au niveau des acides aminés 30 à 36, 39 à 47 ou 260 à 264 de SEQ ID N^{o} 1 en changeant la Ser au niveau de l'acide aminé 32 ou de l'acide aminé 262 en Gly, ou la Tyr au niveau de l'acide aminé 42 en Phe.

12. Protéine ayant une activité d'IkB qui comprend la séquence d'acides aminés allant de la position N^{o} 1 à la position N^{o} 280 de SEQ ID N^{o} 1, ou une de ses parties qui a une activité d'IkB, et qui comprend une séquence signal spécifique de noyau hétérologue.

13. Cellules endothéliales non humaines selon la revendication 1 ou 2, ou tissus ou organes donneurs selon la revendication 3 ou 4, exprimant un ou plusieurs des facteurs de restriction de complément humains choisis parmi le facteur I, le facteur H, la protéine de liaison C4, DAF, la protéine cofacteur membranaire, CR1, CR2, C8bp, P-18 ou SP4040.
